# EUROPÄISCHE PATENTSCHRIFT

(11) **EP 0 496 854 B1**
(45) Veröffentlichungstag und Bekanntmachung des Hinweises auf die Patenterteilung: **15.03.2000**
(21) Anmeldenummer: 91914058.2
(22) Anmeldetag: 01.08.1991
(51) Int. Cl.: C07J 9/00, C07J 17/00, C12P 33/00

(54) **Verfahren zur Herstellung von 20-METHYL-5,7-PREGNADIEN-3$g(b),21-DIOL-DERIVATIVEN**
Method for preparation of 20-METHYL-5,7-PREGNADIENE-3$g(b),21-DIOL DERIVATIVES
Procédé pour la preparation des DERIVES DE 20-METHYLE-5,7-PREGNADIENE-3$g(b),21-DIOL

(30) Priorität: 18.08.1990 DE 4026463
(43) Veröffentlichungstag der Anmeldung: 05.08.1992
(73) Patentinhaber: SCHERING AKTIENGESELLSCHAFT, 13342 Berlin (DE)
(72) Erfinder: WEBER, Alfred, D-1000 Berlin 37 (DE); KENNEKKE, Mario, D-1000 Berlin 33 (DE); NEEF, Günter, D-1000 Berlin 31 (DE)
(86) Internationale Anmeldenummer: DE9100623
(87) Internationale Veröffentlichungsnummer: WO9203465

(56) Entgegenhaltungen:
- EP-A- 0 045 524
- EP-A- 0 377 743
- US-A- 4 214 052
- Tetrahedron Letters, Band 21, Nr. 52, 1980, Pergamon Press Ltd (GB), H. Takayama et al.: "Facile, stereoselective synthesis of (24R)-24,25-dihydroxyvitamin D3 using D-glyceric acid as a chiral synthon", Seiten 5027-5028, siehe den ganzen Artikel
- Chemical Abstracts, Band 98, Nr. 3, 17. Januar 1983, (Columbus, Ohio, US), siehe Seite 536, Zusammenfassung 16936u, & JP, A, 57106699 (CHUGAI PHARMACEUTICAL CO. LTD) 2. Juli 1982

## Beschreibung

Die Erfindung betrifft ein Verfahren zur Herstellung von 20-Methyl-5,7-pregnadien-3β,21-diol-Derivaten der allgemeinen Formel I 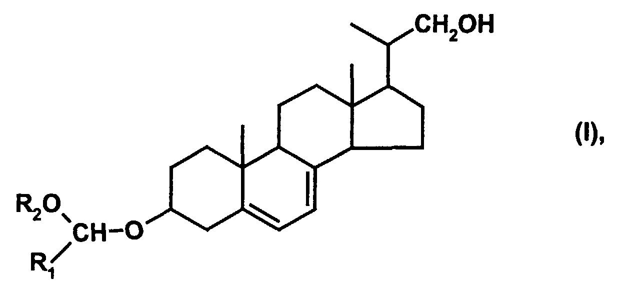 worin
R₁ ein Wasserstoffatom und
R₂ eine Methylgruppe bedeuten oder
R₁ und R₂ gemeinsam eine Tetramethylengruppe oder eine Pentamethylengruppe darstellen, welches dadurch gekennzeichnet ist, daß man ein Ergosterin-Derivat der allgemeinen Formel II 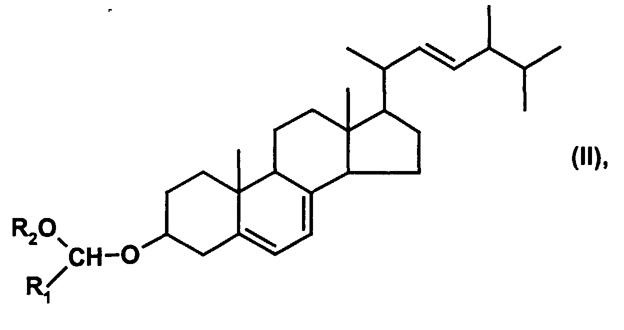 worin R₁ und R₂ die in Formel I genannte Bedeutung besitzen, mit einer Bakterienkultur der Spezies Mycobacterium spec. NRRL B-3683, Mycobacterium spec. NRRL B-3805, Mycobacterium phlei NRRL B-8154 oder Mycobacterium fortuitum NRRL B-8153 fermentiert.

US-A-4,214,052 offenbart ein mikrobiologische Verfahren zur Herstellung von 9α-OH-Steroiden unter Verwendung von Mikroorganismen.

Es ist für den Fachmann sehr überraschend, daß unter den Bedingungen des erfindungsgemäßes Verfahrens die 20-Methyl-5,7-pregnadien-3β,21-diol-Derivate der allgemeinen Formel I gebildet werden, denn es ist bekannt, daß andere Sterin-3-acetale unter diesen Bedingungen zu 17-Oxosteroiden abgebaut werden (US-Patent 4,179,336).

Das erfindungsgemäße Verfahren wird unter den gleichen Fermentationsbedingungen durchgeführt, welche man auch bei den bekannten mikrobiologischen Umwandlungen von Substraten mit diesen Bakterienkulturen anwendet.

Unter den für diese Bakterienkulturen üblicherweise verwendeten Kulturbedingungen werden in einem geeigneten Nährmedium unter Belüften Submerskulturen angezüchtet. Dann setzt man den Kulturen das Substrat (in einem geeigneten Lösungsmittel gelöst oder in emulgierter Form) zu und fermentiert, bis eine maximale Substratumwandlung erreicht ist.

Geeignete Substratlösungsmittel sind beispielsweise Methanol, Ethanol, Glykolmonomethylether, Dimethylformamid oder Dimethylsulfoxyd. Die Emulgierung des Substrats kann beispielsweise bewirkt werden, indem man dieses in mikronisierter Form oder in einem mit Wasser mischbaren Lösungsmittel (wie Methanol, Ethanol, Aceton, Glykolmonomethylether, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Ethylenoxyaddukte oder Fettsäureester von Polyglykolen. Als geeignete Emulgatoren seien die handelsüblichen Netzmittel Tegin®, Tween® und Span® beispielsmäßig genannt.

Die optimale Substratkonzentration, Substratzugabezeit und Fermentationsdauer ist von der Art des verwendeten Substrates und Mikroorganismus und den Fermentationsbedingungen abhängig. Diese Größen müssen, wie dies bei mikrobiologischen Steroidumwandlungen allgemein erforderlich ist, im Einzelfall durch Vorversuche, wie sie dem Fachmann geläufig sind, ermittelt werden.

Die nach dem erfindungsgemäßen Verfahren hergestellten 20-Methyl-5,7-pregnadien-3β,21-diol-Derivate der allgemeinen Formel I sind wertvolle Zwischenprodukte, welche vorzugsweise zur Synthese von Vitamin D₃ und seinen Derivaten verwendet werden können. So kann man beispielsweise diese Verbindungen zu den 21-Tosylaten verestern und diese unter den Bedingungen die A. Fürst et. al. beschrieben haben (Helv. Chim. Acta 65, 1982, 1499-1521) umsetzen. Man erhält so Verbindungen der allgemeinen Formel III 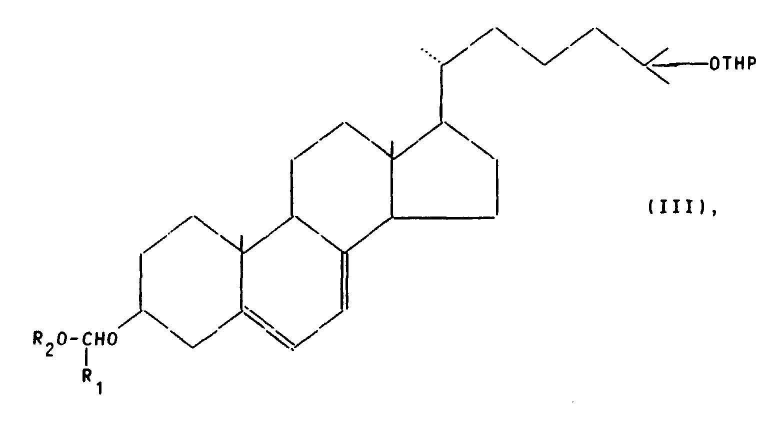 deren Acetalgruppen abgespalten werden. Die fotochemische Umlagerung des so erhaltenen 25-Hydroxycholesterins in 25-Hydroxy-vitamin D₃ kann ebenfalls nach dem Verfahren durchgeführt werden, wie sie von A. Fürst et. al. beschrieben wurden.

Die Ausgangsverbindungen für das erfindungsgemäße Verfahren können unter den im US-Patent 4,179,336 beschriebenen Bedingungen hergestellt werden.

Das nachfolgende Ausführungsbeispiel dient zur näheren Erläuterung des erfindungsgemäßen Verfahrens.

### Beispiel

a) 400 g Ergosterin werden in 6 l Formaldehyddimethylacetal gelöst, unter Rühren bei Raumtemperatur mit 400 g Kieselgur und portionsweise mit 200 g Phosphorpentoxid versetzt und 2 Stunden bei Raumtemperatur gerührt. Es wird vom Unlöslichen abfiltriert, mit Formaldehyddimethylacetal gewaschen und das Lösungsmittel im Vakuum abdestilliert. Nach Zugabe von Natriumhydrogencarbonatlösung wird das feste Rohprodukt abfiltriert, mit Wasser gewaschen und man erhält nach Trocknung 440 g 3β-Methoxymethoxy-5,7,22-ergostatrien.
b) Ein 2 l Erlenmeyer mit 500 ml sterilem Nährmedium enthaltend
   1 % Hefeextrakt
   0,45 % Na₂HPO₄
   0,34 % KH₂PO₄
   0,2 % Tween 80
   -eingestellt auf pH 6,7-
   wird mit einer Abschwemmung einer Trockenkultur von Mycobacterium spec. NRR1 B-3805 beimpft und 3 Tage mit 180 Umdrehungen bei 30° C geschüttelt.
c) 22,5 g 3β-Methoxymethoxy-ergosta-5,7,22-trien werden mit 4,4 g Tegin und 430 ml Wasser bei 95° C mit einem Ultra-Turrax (Firma Jahnke & Kunkel) 25 Minuten emulgiert und anschließend auf 1000 g mit Wasser aufgefüllt. Man sterilisiert die Emulsion 20 Minuten lang bei 120° C.
d) Ein 500 ml Erlenmeyer mit 100 ml sterilem Nährmedium enthaltend
   2,5 % Cornsteep liquor
   0,25 % Sojamehl
   0,3 % (NH₄)₂HPO₄
   0,25 % Tween 80
   -eingestellt auf pH 6,5-
   wird mit 5 ml der Mycobacterium spec. Anzuchtskultur beimpft. Dann werden 13,3 ml der unter b) hergestellten Emulsion (dies entspricht 0,3 g 3β-Methoxymethoxy-ergosta-5,7,22-trien) zugegeben und 120 Stunden lang bei 30° C unter Schütteln mit 220 Umdrehungen pro Minute fermentiert.

Nach erfolgter Fermentation wird die Kulturbrühe zweimal mit je 100 ml Methylisobutylketon extrahiert. Die vereinigten Extrakte werden sodann mit 11 g Aktivkohle versetzt und über ein Faltenfilter filtriert. Das Filtrat wird anschließend bei 50° C im Rotationsverdampfer eingeengt und an Aluminiumoxid chromatographiert. Nach erfolgter Chromatographie erhält man 48 mg 3β-Methoxymethoxy-20-methyl-5,7-pregnadien-21-ol, welches nach HPLC mit einer authentischen Probe identisch ist.

## Patentansprüche

1. Verfahren zur Herstellung von 20-Methyl-5,7-pregnadien-3β,21-diol-Derivaten der allgemeinen Formel I 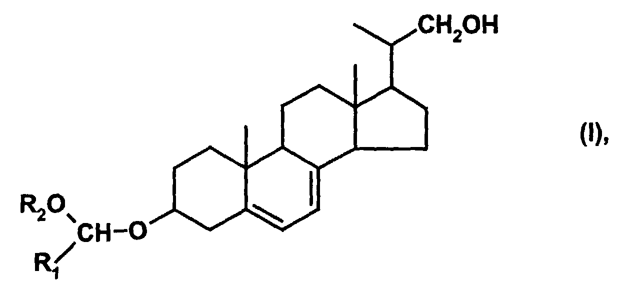 worin
R₁ ein Wasserstoffatom und
R₂ eine Methylgruppe bedeuten oder
R₁ und R₂ gemeinsam eine Tetramethylengruppe oder eine Pentamethylengruppe darstellen, dadurch gekennzeichnet, daß man ein Ergosterin-Derivat der allgemeinen Formel II 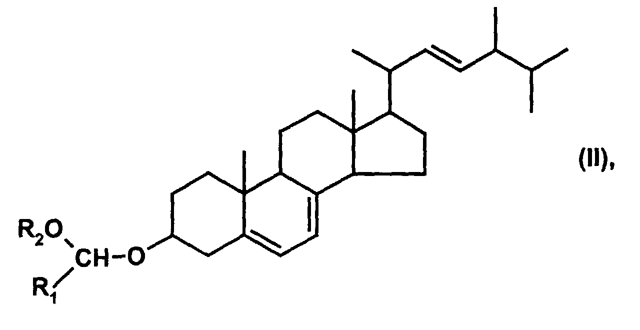 worin R₁ und R₂ die in Formel I genannte Bedeutung besitzen, mit einer Bakterienkultur der Spezies Mycobacterium spec. NRRL B-3683, Mycobacterium spec. NRRL B-3805, Mycobacterium phlei NRRL B-8154 oder Mycobacterium fortuitum NRRL B-8153 fermentiert.

## Claims

1. Process for preparing 20-methyl-5,7-pregnadiene-3β,21-diol derivatives of the general formula I 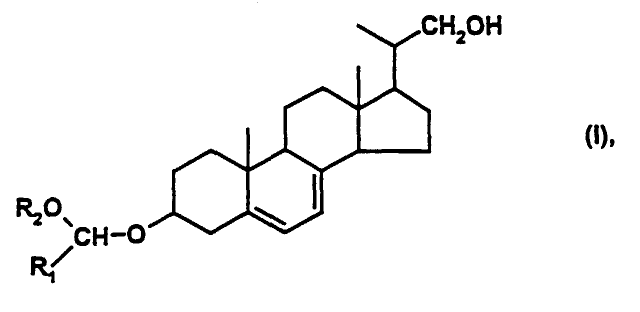 in which
R₁ is a hydrogen atom and
R₂ is a methyl group or
R₁ and R₂ together are a tetramethylene group or a pentamethylene group,
characterized in that an ergosterol derivative of the general formula II 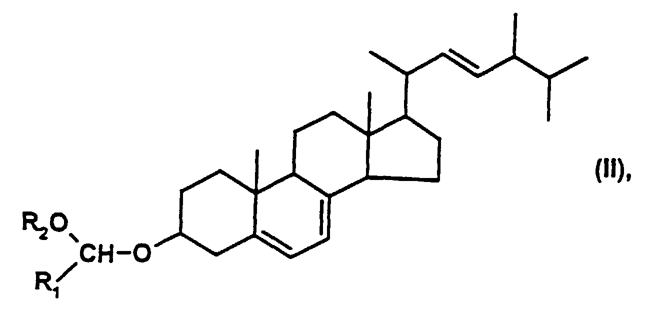 in which R₁ and R₂ are as defined in formula I are fermented with a bacteria culture of the species Mycobacterium spec. NRRL B-3683, Mycobacterium spec. NRRL B-3805, Mycobacterium phlei NRRL B-8154 or Mycobacterium fortuitum NRRL B-8153.

## Revendications

1. Procédé de préparation de dérivés de 20-méthyl-5,7-prégnadiène-3β,21-diol de formule générale I 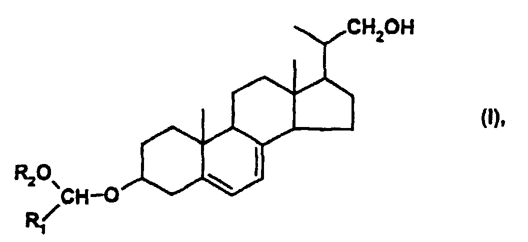 dans laquelle
R₁ signifie un atome d'hydrogène et
R₂ signifie un groupement méthyle ou
R₁ et R₂ représentent ensemble un groupement tétraméthylène ou un groupement pentaméthylène, caractérisé en ce qu'on fermente un dérivé d'ergostérol de formule générale II 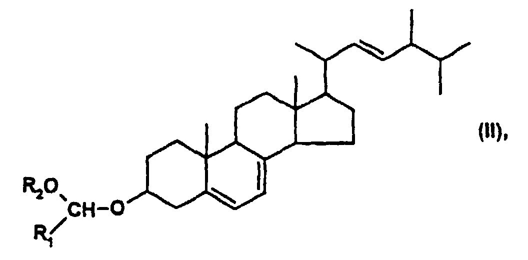 dans laquelle R₁ et R₂ qui possèdent la signification citée à la formule I fermentent avec une culture bactérienne du genre Mycobacterium species NRRL B-3683, Mycobacterium species NRRL B-3805, Mycobacterium NRRL B-8154 ou Bacterium fortuitum NRRL B-8153.
